(19) Europäisches Patentamt
European Patent Office
Office européen des brevets

(11) Numéro de publication: **0 421 848 B1**

(12) **FASCICULE DE BREVET EUROPEEN**

(45) Date de publication de fascicule du brevet: **08.02.95**

(51) Int. Cl.$^6$: **C07K 1/02**, C07K 1/06, C07K 17/06

(21) Numéro de dépôt: **90402677.0**

(22) Date de dépôt: **28.09.90**

# (54) Procédé de solubilisation de peptides et procédé de synthèse de peptides.

(30) Priorité: **02.10.89 FR 8913054**

(43) Date de publication de la demande:
**10.04.91 Bulletin 91/15**

(45) Mention de la délivrance du brevet:
**08.02.95 Bulletin 95/06**

(84) Etats contractants désignés:
**AT BE CH DE DK ES FR GB GR IT LI LU NL SE**

(56) Documents cités:

**CHEMICAL ABSTRACTS, vol. 89, no. 9, 28 août 1978, Columbus, OH (US); p. 415, no. 75456z&NUM;**

**CHEMICAL ABSTRACTS, vol. 91, no. 19, 05 novembre 1979, Columbus, OH (US); H. ANZINGER et al., p. 673, no. 158070m&NUM;**

(73) Titulaire: **RHONE-POULENC CHIMIE**
**25, Ouai Paul Doumer**
**F-92408 Courbevoie Cédex (FR)**

(72) Inventeur: **Gervais, Christian**
**6, allée Marcel Achard**
**F-69100 Villleurbanne (FR)**
Inventeur: **Bernard, Jean-Marie**
**Route du Large,**
**Saint Laurent d'Agny**
**F-69440 Mornant (FR)**
Inventeur: **Bouzid, Kamel**
**45, rue du Lieutenant Colonel Girard**
**F-69007 Lyon (FR)**

(74) Mandataire: **Ricalens, François et al**
**RHONE-POULENC CHIMIE,**
**Direction de la Propriéte Industrielle,**
**25, quai Paul Doumer**
**F-92408 Courbevoie Cédex (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services
(3.10/3.09/3.3.3)

## Description

La présente invention a pour objet un procédé de solubilisation de peptides. Elle a aussi pour objet un procédé de synthèse de peptides en phase liquide.

Il existe un grand nombre de méthodes de synthèse de peptides décrites dans la littérature. Elles ont toutes des points communs de base qui consistent :

- à protéger la fonction de la chaine latérale d'un aminoacide par un groupe protecteur clivable en fin de synthèse du peptide,
- à protéger la fonction amide (N$\alpha$) dudit aminoacide par un groupement protecteur clivable après la condensation de l'aminoacide,
- à activer la fonction acide carboxylique dudit aminoacide protégé puis à le condenser avec un acide aminé ou un peptide dont la fonction C terminale est protégée et dont la fonction amine est libre,
- le peptide est obtenu par déprotection totale des groupes protecteurs après la condensation de tous les acides aminés.

Les diverses méthodes de synthèse se distinguent par l'état physique de la phase où a lieu la synthèse : phase liquide ou phase solide.

Les méthodes de synthèse dites en phase liquide consistent à réaliser toutes les réactions en phase homogène.

Dans la méthode décrite par Bodansky et du Vigneaud dans le Journal of American Chemical Society 81, 5688-5691 (1959) l'aminoacide de départ est protégé par un groupe méthylique et les aminoacides successifs sont condensés "pas à pas" après protection de leur fonction amine par un groupe benzyl oxycarbonyle et activation de leur fonction carboxylique par un ester nitrophénylique. Les intermédiaires successifs sont purifiés par précipitation ou lavages. Cette technique requiert un nombre élévé d'étapes de synthèse délicates ou longues qui entraînent inévitablement une perte importante de produits ; c'est ainsi que Schwyzer et Sieber (Helvetica Chemica Acta, 49, 134-158, 1966) obtiennent par cette technique un rendement pour l'ACTH de seulement 6,85 %.

La synthèse décrite par Beyermann et Coll (Rec. Trav. Chim. Pays Bas 92, 481, 1973) consiste à appliquer la même méthode que précédemment, à partir d'un aminoacide ou d'un peptide dont le groupement carboxylique est protégé par un groupe benzylique et à réaliser les couplages en présence d'un excès d'anhydride d'aminoacide protégé, de façon à augmenter les rendements. Le nombre d'opérations est aussi élevé que dans le procédé précédent et le peptide formé perd souvent sa solubilité en milieu organique dès que le nombre d'aminoacides dépasse quatre ou cinq.

Certaines méthodes de synthèse en phase liquide utilisent des agents protecteurs solubilisants de l'aminoacide ou du peptide de départ plus sophistiqués ; ainsi le brevet européen n° 0 017 536 mentionne comme groupe protecteur le groupe phénylazobenzyl sulfonyléthyloxy (OPSE). Ce groupe permet la solubilisation des peptides formés dans le diméthyl formamide et leur insolubilisation, tant dans l'eau que dans les autres solvants organiques. La synthèse se fait toujours "pas à pas" avec précipitation de chaque intermédiaire peptidique formé avant l'ajout d'un nouvel aminoacide, ce qui pose des problèmes au niveau de la filtration des solides. Ce procédé comme l'ensemble des procédés précédemment évoqués ne permet pas de maintenir en solution des peptides ayant un grand nombre d'aminoacides.

Parmi les procédés en phase liquide, on peut encore citer les procédés dans lesquels l'aminoacide est maintenu en solution par l'usage d'un polymère linéaire non réticulé, tel que le polyéthylèneglycol (Mutter et Bayer, Nature 237, 512 (1972), les sous-produits étant éliminés par ultracentrifugation ou par précipitation.

Parmi les méthodes de synthèse dites en phase solide, on peut citer la technique décrite par Merrifield en 1963 dans le Journal of American Chemical Society 85, 2149-2154, qui consiste à fixer la fonction carboxylique C-terminale du premier aminoacide ou du premier groupe peptidique sur un support insoluble. Les couplages et les lavages peuvent être standardisés de façon à rendre le procédé automatisable. Cette technique reste à ce jour encore fort coûteuse, car il est nécessaire d'utiliser les réactifs en grand excès de façon à convertir toutes les chaines peptidiques en cours de synthèse dans un temps raisonnable. La pureté et l'homogénéité des peptides formés est encore loin d'être idéale, les sous-produits constitués de chaînes peptidiques non complètes doivent être éliminés en fin de réaction. En effet, la purification au stade intermédiaire est impossible, tandis qu'au stade final elle est difficile, car elle demande un appareillage compliqué et une technique sophistiquée, donc coûteuse telle que la chromatographie liquide haute pression préparative.

L'extrapolation de cette technologie au stade industriel reste un problème non résolu à ce jour, en raison des difficultés de mise en oeuvre de fortes quantités de résines, soit en réacteur agité, soit en lit fixe.

La présente invention a permis de résoudre une grande partie des problèmes laissés en suspens dans l'art antérieur.

Il a maintenant été trouvé un procédé de solubilisation de peptides qui peut permettre leur synthèse en phase liquide non aqueuse et leur purification.

Le procédé de solubilisation de peptides éventuellement protégés ou salifiés, dans un solvant organique non miscible à l'eau, est caractérisé en ce que le groupe C-terminal dudit peptide est lié par l'intermédiaire d'une liaison amide ou ester à un groupe lipophile, ce dernier lorsqu'il est lié à la L-sérine donnant une molécule dont la solubilité dans l'eau à 25 °C est inférieure à 30 g/litre, ce groupe lipophile étant chimiquement défini et non polymérique.

Le groupe lipophile, qui va permettre la synthèse peptidique en phase liquide organique non miscible à l'eau, est de préférence composé de deux entités A et L liées entre elles par une liaison covalente et liées au peptide de façon covalente par l'intermédiaire de l'entité A :

Peptide - CO - A - L

L'entité - A - représente un bras d'ancrage bifonctionnel entre le peptide en cours de synthèse et le groupe L.

Le groupe L représente la partie lipophile de l'entité A - L. Ce groupe L n'est en aucun cas polymérique. C'est de préférence un groupe hydrocarboné comportant éventuellement des halogènes ; il répond à la formule générale :

$- C_{(a)} H_{(b)} O_{(c)} N_{(d)} S_{(e)} Si_{(f)} X_{(g)}$

dans laquelle:

- a est un nombre entier compris entre 1 et 50
- b est un nombre entier compris entre 3 et 101
- c est un nombre entier compris entre 0 et 6
- d est un nombre entier compris entre 0 et 2
- e est un nombre entier compris entre 0 et 2
- f est un nombre entier compris entre 0 et 2
- g est un nombre entier compris entre 0 et 20
- X représente un halogène choisi parmi le fluor, le chlore et le brome.

On préfère parmi l'ensemble des composés pouvant représenter le groupe L ceux pour lesquels (a) représente un nombre entier supérieur à 6 et encore plus préférentiellement supérieur à 12, ainsi que ceux pour lesquels (g) représente un nombre entier supérieur à 2.

Le groupe A qui, comme précisé précédemment représente le bras d'ancrage bifonctionnel, comporte à une de ses extrémités au moins une fonction alcool ou amine (B) et à l'autre extrémité une fonction carbonyle ou une fonction éther. Il peut être représenté par la formule générale suivante :

$$B - R_1 - Ar_{(k)} - \left[ R_2 - \underset{\displaystyle O}{\overset{\|}{C}}{}_{(n)} - O_{(m)} \right]_p$$

dans laquelle :

- B représente un groupe hydroxyle ou amino
- Ar représente un radical aromatique mono ou polycyclique
- $R_1$ représente une liaison covalente, un radical alkylène contenant 1 à 4 atomes de carbone éventuellement substitué par un radical phényle, un groupe alkylènecarbonyle
- $R_2$ représente une liaison covalente, un radical alkylène contenant 1 à 4 atomes de carbone éventuellement substitué, un groupe oxyalkylène contenant 1 à 4 atomes de carbone dans la chaîne alkylène, un atome d'oxygène
- k, m et n sont des nombres entier égaux à 0 ou 1
- p est un nombre entier égal à 1 ou 2.

Il est évident à la lecture de la présente description que les deux entités A et L peuvent former un seul groupe répondant aux formules :

$B-R_1-Ar'$ ou $B-CH_{(3-q)}-Ar''_{(q)}$

dans lesquelles :

- B et $R_1$ ont les mêmes significations que précédemment
- Ar' représente un radical aromatique polycyclique
- Ar'' représente un radical benzénique et q est un nombre entier égal à 2 ou 3.

On peut citer parmi les groupes A - les groupes de formules suivantes :

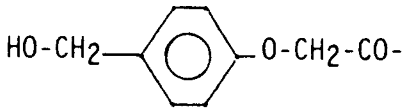

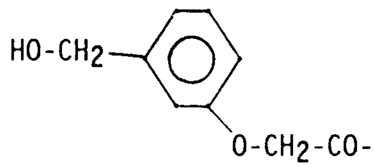

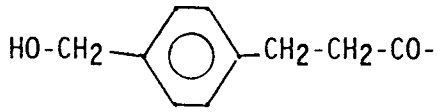

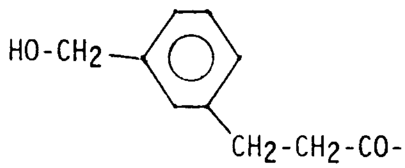

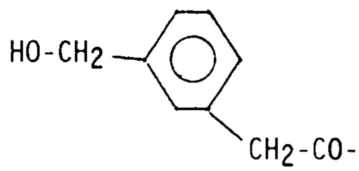

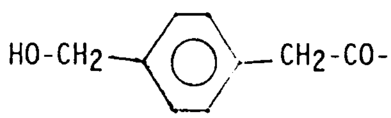

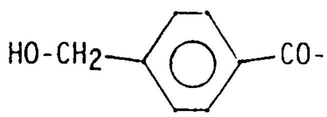

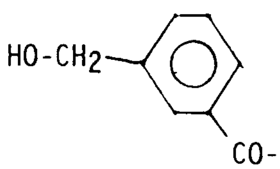

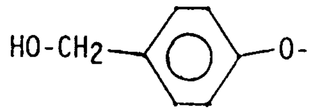

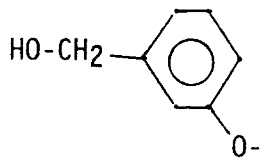

$HO-CH_2-CO-O-$

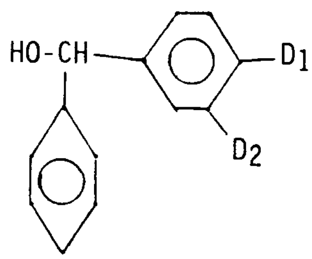

dans laquelle :

- $D_1$ et $D_2$ représentent chacun un groupe identique ou différent choisi parmi les radicaux ou atomes O ; CO ; $CH_2O$ ; $CH_2CO$ ; OCO
- $D_1$ ou $D_2$ représente H ;

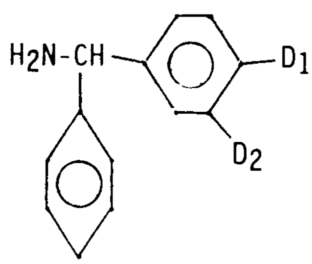

dans laquelle $D_1$ et $D_2$ ont les mêmes significations que précédemment ;

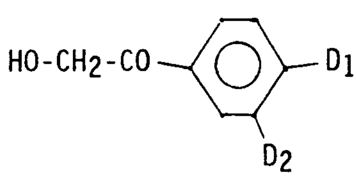

dans laquelle $D_1$ et $D_2$ ont les mêmes significations que précédemment.

On peut citer parmi les groupes lipophiles - L les groupes de formules suivantes :

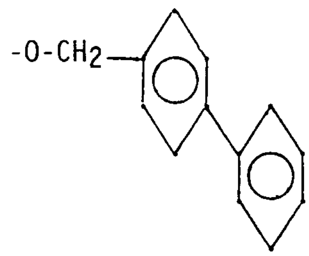

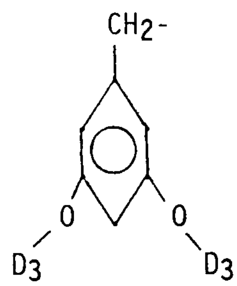

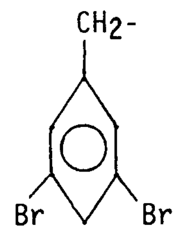

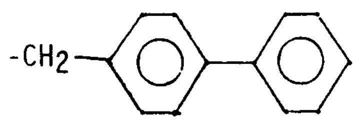

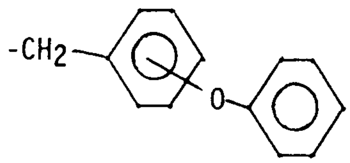

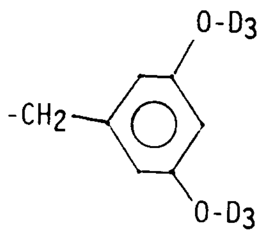

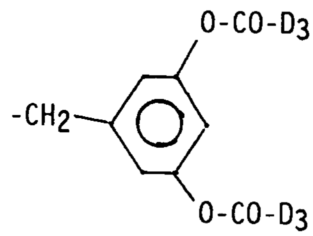

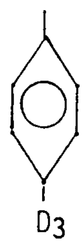

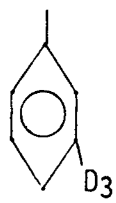

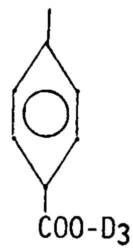

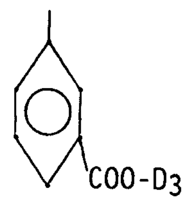

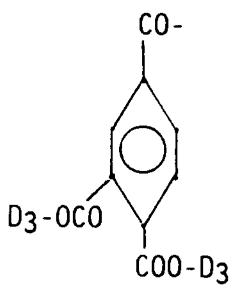

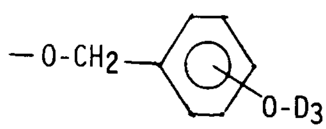

dans lesquelles $D_3$ représente un radical alkyle ayant 1 à 12 atomes de carbone ;

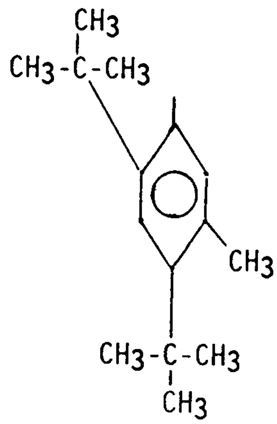

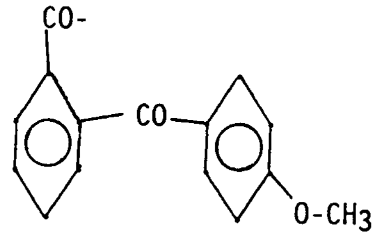

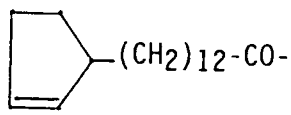

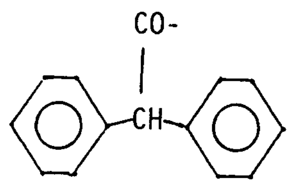

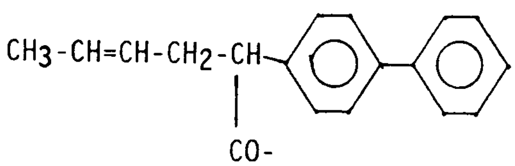

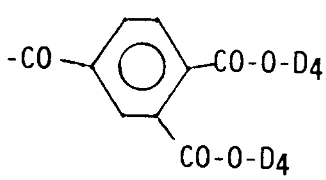

dans laquelle $D_4$ représente un groupe aryle ou aralkyle ;

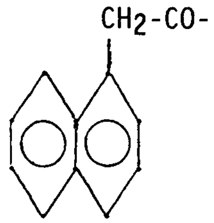

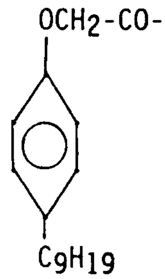

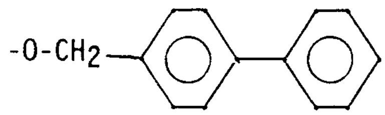

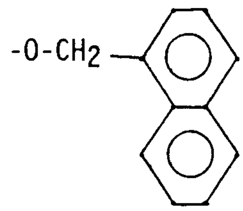

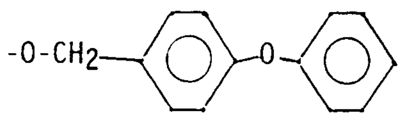

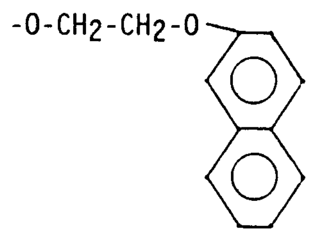

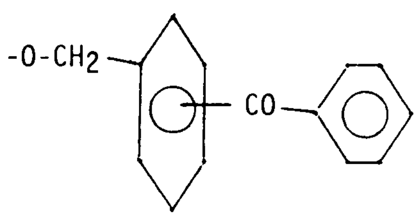

Les solvants dans lesquels les peptides sont solubilisés grâce à leur liaison au groupe lipophile sont des solvants organiques non miscibles à l'eau, tels que les dérivés aliphatiques halogénés en particulier le chlorure de méthylène, les dérivés aromatiques tels que l'anisole ou le chlorobenzène, les esters tels que l'acétate d'éthyle.

Le procédé de solubilisation permet d'augmenter notablement la solubilité des peptides en phase organique. Il permet par exemple d'obtenir des solutions homogènes de concentration supérieure ou égale à 50 g/litre.

Un autre avantage du présent procédé provient de ce que les peptides liés aux groupements A - L définis précédemment ont un coefficient de partage élevé en faveur de la phase organique lors de la décantation en présence d'eau.

Cette propriété peut être mise à profit pour purifier les peptides par des lavages aqueux.

La présente invention concerne aussi un procédé de synthèse de peptides éventuellement protégés, en milieu liquide, caractérisé en ce que l'on part d'un aminoacide ou d'un peptide solubilisé en milieu

organique par un groupe A - L tel que défini précédemment, lié à la fonction carboxylique de l'aminoacide ou du peptide de départ et que l'on ajoute les aminoacides ou les peptides à condenser activés sur leur fonction acide et protégés sur leur fonction amine et éventuellement sur leur chaîne latérale.

La protection de la fonction amine des aminoacides peut s'effectuer par remplacement d'un atome d'hydrogène de cette fonction par un groupe tel que le tertiobutyloxycarbonyle ou par la formation de sels de DANE, par exemple par réaction de la fonction amine avec un composé béta-dicarbonylé.

L'activation du peptide ou de l'aminoacide N-protégé est réalisée par formation, soit d'un anhydride mixte au moyen d'un chlorure d'acide, soit d'un anhydride mixte au moyen de chloroformiate d'alkyle, soit d'un anhydride symétrique au moyen d'un carbodiimide, soit d'un ester activé selon une technique classique de synthèse, soit par toute autre technique d'activation d'un aminoacide N-protégé.

Un avantage du présent procédé est qu'il permet une synthèse productive et répétitive, en milieu organique, où le peptide lié à la molécule lipophile A - L définie précédemment, est toujours maintenu en solution. Chaque intermédiaire peptide-A - L, N-protégé ou non est maintenu soluble dans le milieu organique.

Un autre avantage important du procédé est qu'il permet d'éliminer de la phase organique les réactifs en excès et les co-produits de la synthèse, par simple lavage à l'eau :

- après l'étape de condensation, les produits tels que les excès d'aminoacides activés ou de peptides activés, les sels, les acides ou alcools ou tous les autres co-produits de la réaction qui ne sont pas liés à la chaîne peptidique synthétisée ;
- après l'étape de déprotection, les agents de déblocage des fonctions désirées et les co-produits de la réaction de coupure du groupe N-protecteur.

Ce procédé évite ainsi toutes les étapes de purification par précipitation nécessaires dans l'art antérieur.

Le suivi de la pureté du peptide en cours de synthèse est possible à tout moment, par simple prélèvement et analyse par toute technique telle que la chromatographie liquide haute performance, la résonance magnétique nucléaire du proton ou du carbone, la potentiométrie, la spectrométrie de masse ...

Du fait de la similitude des opérations de condensation et de déprotection, lors de l'addition de chacun des acides aminés, le procédé de la présente invention permet la synthèse des peptides selon un mode opératoire répétitif.

Du fait de la répétitivité de la synthèse, le procédé peut être avantageusement mis en oeuvre sous une forme automatisée.

Enfin, lorsque la synthèse de la séquence initiale du peptide est terminée, le peptide est libéré de ses groupes protecteurs et du groupe solubilisant A - L par hydrolyse, hydrogénolyse ou par toute autre méthode de déprotection utilisée en synthèse peptidique.

Ce procédé est particulièrement intéressant pour la synthèse de peptides hydrophiles ou porteurs de groupes hydrophiles, comme par exemple ceux qui comportent dans leur chaîne les acides aminés suivants : l'arginine, la glutamine, l'asparagine, la sérine, la thréonine, la glycine.

Les peptides issus de la synthèse sont utilisés éventuellement pour la synthèse de médicaments, vaccins, produits agro-alimentaires ou phytosanitaires.

La présente invention sera plus complètement décrite à l'aide des exemples suivants qui ne doivent pas être considérés comme limitatifs de l'invention.

## I - SYNTHESE DES REACTIFS PERMETTANT D'OBTENIR LES GROUPES SOLUBILISANTS A-L

### EXEMPLE 1 : Préparation du chlorométhyl-3 benzoate de phényl-4 benzyle

Dans un ballon tricol de 500 $cm^3$, on introduit successivement :

- 40 g (0,217 mol) de biphénylméthanol
- 150 $cm^3$ de toluène
- 42,9 g (0,227 mol) de chlorure de chlorométhyl-3 benzoyle.

Le mélange est agité et chauffé à 40 °C.

On ajoute alors en 10 minutes 25 $cm^3$ de N-méthylmorpholine (0,227 mol).

La température du milieu réactionnel s'élève spontanément à 60 °C, puis on le chauffe à 80 °C pendant 2 heures 30 minutes.

Le mélange réactionnel final est versé dans une ampoule à décanter, dilué à 1 litre par addition d'acétate d'éthyle, puis lavé successivement par 450 $cm^3$ d'acide chlorhydrique normal, 400 $cm^3$ d'une solution aqueuse normale d'hydrogénocarbonate de sodium, puis par de l'eau jusqu'à neutralité.

La distillation des solvants conduit à un résidu solide qui est cristallisé de chaud à froid dans 1,6 litre de méthanol.

Le chlorométhyl-3 benzoate de phényl-4 benzyle est obtenu avec un rendement de 81 % (59,5 g), sous la forme d'un solide blanc ayant un point de fusion de 102 ° C.

Sa structure a été établi par spectrométrie de masse et de RMM du proton (360 MHz).

L'analyse par chromatographie en couche mince (CCM) sur plaque de silice Merck 60F 254 révèle une seule tache dans chacun des deux systèmes d'éluants essayés :

acétate d'éthyle/cyclohexane (2/5) Rf = 0,7

hexane/acétone (4/1) Rf = 0,6.

EXEMPLES 2 à 14

En opérant selon le même mode opératoire que dans l'exemple 1, on prépare les composés suivants.

Exemple 2

Chlorométhyl-3 benzoate de phénoxy-3 benzyle

Aspect : huile
Rendement : 94 %

Exemple 3

Chlorométhyl-3 benzoate de méthylène-9 anthracyle

Aspect : solide ayant un point de fusion de 148 ° C (recristallisation dans le méthanol)
Rendement : 82 %

Exemple 4

Bromométhyl-3 benzoate de phényl-4 benzyle

Aspect : solide ayant un point de fusion de 63-65 ° C
Rendement : 26 %

Exemple 5

Chlorométhyl-3 benzoate de tertiobutyl-4 benzyle

Aspect : huile
Rendement : 47 %

Exemple 6

Chlorométhyl-3 benzoate de dichloro-2,4 benzyle

Aspect : solide ayant un point de fusion de 75 ° C (recristallisation dans acétone + eau)
Rendement : 75 %

Exemple 7

Chlorométhyl-3 benzoate de cholestéryle

Aspect : solide ayant un point de fusion de 122 ° C (recristallisation dans dichlorométhane + méthanol)
Rendement : 78 %

Exemple 8

Chlorométhyl-3 benzoate de phytyle

Aspect : huile
Rendement : 53 %

Exemple 9

Chlorométhyl-3 benzoate d'heptafluoro-2,2,3,3,4,4,4 butyle

Aspect : huile
Rendement : 79 %

Exemple 10

Chlorométhyl-3 benzoate de pentadécafluoro-2,2,3,3,4,4,5,5,6,6,7,7,8,8,8 octyle

Aspect : huile
Rendement : 81 %

Exemple 11

Chloroacétate de cholestéryle

Aspect : cristaux
Rendement : 72 %

Exemple 12

Chloroacétate d'heptafluoro-2,2,3,3,4,4,4 butyle

Aspect : liquide de point d'ébullition 80°C (sous 2660 Pa)
Rendement : 20 %

Exemple 13

Chloroacétate de pentadécafluoro-2,2,3,3,4,4,5,5,6,6,7,7,8,8,8 octyle

Aspect : huile
Rendement : 72 %

Exemple 14

Chloroacétate de (dichloro-3',5' phénoxy)-4 benzyle

Aspect : huile
Rendement : 97 %

II - SYNTHESE DE DIFFERENTS AMINOACYL-A-L OU PEPTIDE-A-L N-PROTEGES

Exemple 15

Synthèse de l'ester de (méthyl-3 benzoate d'hydroxyméthyl-4 biphényle) de la N-tertiobutyloxycarbonyl-L-leucine.

Dans un ballon tricol de 500 $cm^3$, on introduit successivement :

- 15 g du sel de potassium de la N-tertiobutyloxycarbonyl-L-leucine (soit 0,056 mole),

- 180 $cm^3$ de DMF sec,
- 14,905 g (soit 0,044 mol) de chlorométhyl-3-benzoate d'hydroxyméthyl-4-biphényle en solution dans 100 $cm^3$ de DMF sec et
- 1,5 g de iodure de sodium.

Le mélange est porté, sous agitation, à 75 °C.

Après 2 heures de réaction, il est ajouté 490 $cm^3$ d'acétate d'éthyle.

La solution organique est lavée par successivement deux fois 100 $cm^3$ d'eau, 200 $cm^3$ d'une solution diluée de $KHCO_3$ (pH 8,5), 200 $cm^3$ d'eau, 200 $cm^3$ d'une solution de $KHSO_4$ (pH 2), 200 $cm^3$ d'eau.

La phase organique est séchée sur $Na_2SO_4$, puis filtrée et évaporée pour donner une huile avec un rendement égal à 100 %.

La structure du produit a été confirmée par spectrométrie de masse et par RMN du proton (360 MHz)

L'analyse par chromatographie sur couche mince sur plaque de silice révèle une seule tache dans le système d'élution suivant : acétate d'éthyle/hexane (2/5) Rf = 0,66.

EXEMPLES 16 à 22

En opérant selon le même mode opératoire que dans l'exemple 15, on prépare les composés suivants.

Exemple 16

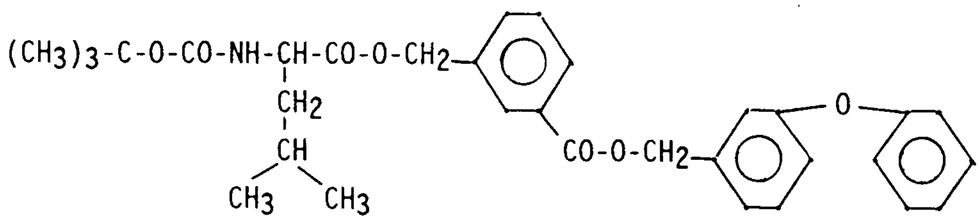

Aspect : huile
Rendement : 98 %

Exemple 17

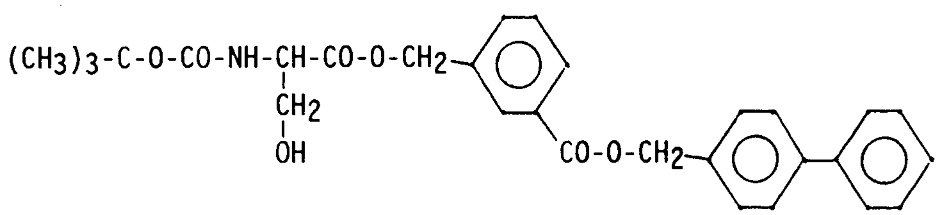

Aspect : huile
Rendement : 95 %

Exemple 18

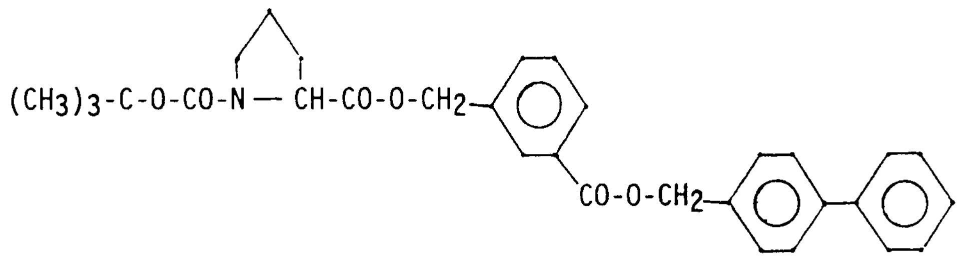

Aspect : huile
Rendement : 100 %

Exemple 19

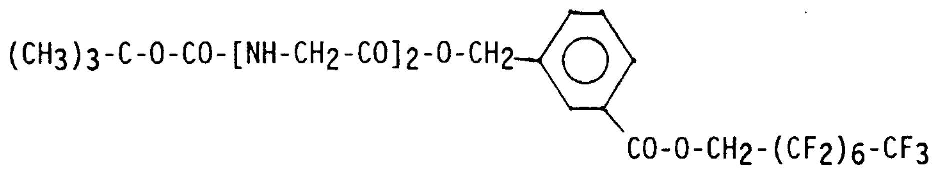

Aspect : poudre blanche
Rendement : 100 %

Exemple 20

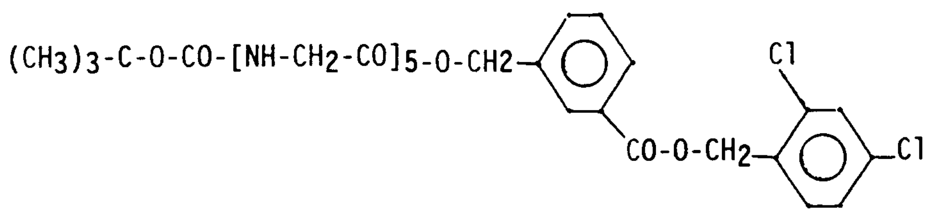

Aspect : poudre blanche
Rendement : 77,5 %

Exemple 21

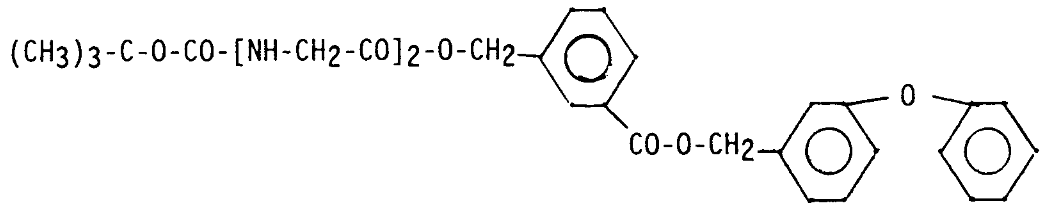

Aspect : huile
Rendement : 100 %

Exemple 22

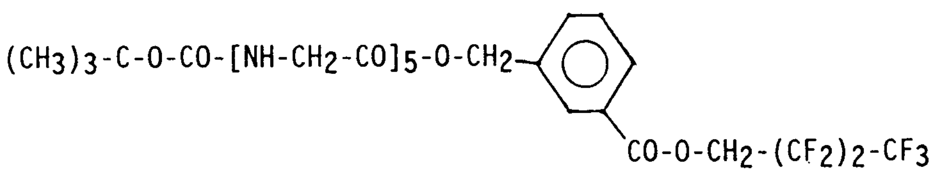

Aspect : poudre
Rendement : 70 %

Exemple 23

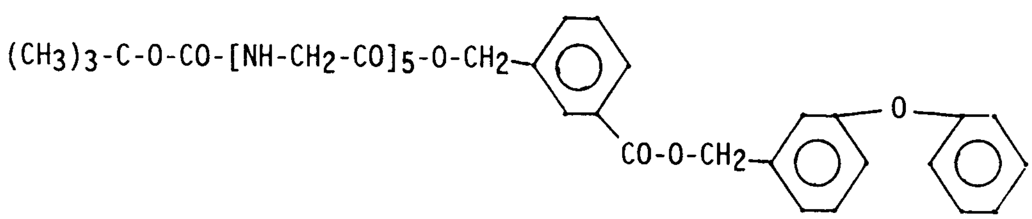

Aspect : poudre blanche
Point de fusion : 215 ° C

Exemple 24

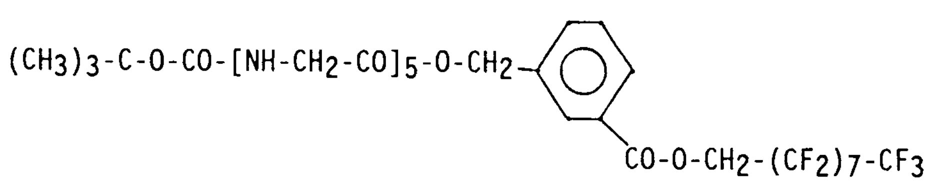

Aspect : poudre blanche
Point de fusion : 240 ° C

Exemple 25

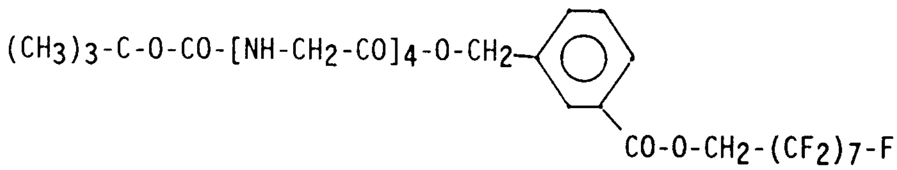

Aspect : poudre blanche

III - SYNTHESE DE DIFFERENTS AMINOACYL-A-L

Exemple 26

Synthèse du chlorhydrate de l'ester de (méthyl-3 benzoate d'hydroxyméthyl-4 biphényle) de la L-leucine.

Dans un ballon tricol de 250 $cm^3$, on introduit successivement :

- 24,6 g (soit 0,044 mol) de l'ester de méthyl-3-benzoate d'hydroxyméthyl-4-biphényle de la N-tertiobutyloxycarbonyl-L-leucine,
- 100 g de dichlorométhane.

Puis on fait barboter un courant d'acide chlorhydrique sec dans le mélange agité pendant 1 heure 30 minutes à température ambiante.

La solution est ensuite dégazée sous courant d'azote pendant 20 minutes.

Le mélange réactionnel est ensuite évaporé jusqu'à un volume de 50 $cm^3$, puis on précipite par addition de 200 $cm^3$ d'éther éthylique.

Le produit obtenu après filtration pèse 19,3 g (0,0413 mol) (rendement 94 %). C'est une poudre blanche ayant un point de fusion de 121 °C - 122 °C.

La structure du produit a été confirmée par spectrométrie de masse et par RMN du proton (360 MHz)

L'analyse par chromatographie sur couche mince sur plaque de silice révèle une seule tache dans le système d'éluants suivant : dichlorométhane/méthanol/acide acétique (90/10/5) Rf = 0,55.

EXEMPLES 27 à 29

En opérant selon le même mode opératoire que dans l'exemple 26, on prépare les composés suivants.

Exemple 27

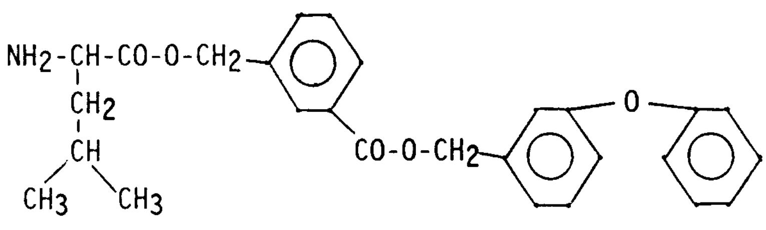

Aspect : huile
Rendement : 98 %

Exemple 28

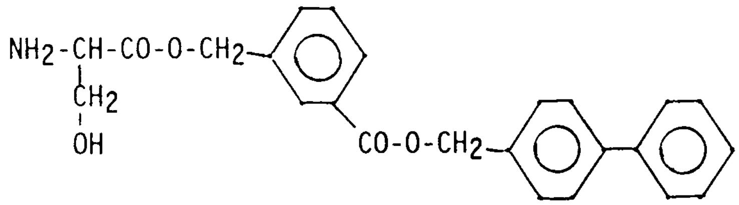

Aspect : poudre blanche
Rendement : 98 %

Exemple 29

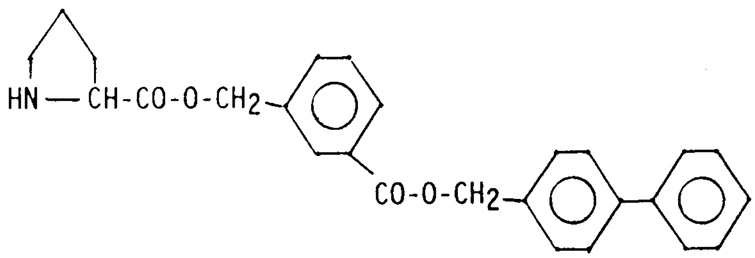

Aspect : poudre blanche
Rendement : 80 %

IV - MESURE DES SOLUBILITES DES MOLECULES DE FORMULE (L)-SERYL-A-L DANS L'EAU

Exemple 30

Les groupes lipophiles A-L lorsqu'ils sont liés à la L-sérine donnent une molécule dont la solubilité dans l'eau est inférieure à 30 g/l à températue ambiante.

Synthèse des molécules de formule (L)-séryl-A-L

Les chlorhydrates de formule HCl, (L)-séryl-A-L obtenus selon la méthode décrite dans les exemples 26 à 29, sont mis en solution dans le dichlorométhane. On fait passer du gaz ammoniac dans cette solution à température ambiante. Le précipité de chlorure d'ammonium obtenu est rapidement filtré. Le filtrat est ensuite concentré sous pression très réduite. Le produit obtenu après concentration est immédiatement trituré dans l'eau pendant 15 min à température ambiante, puis filtré.

La solubilité du produit de formule (L)-séryl-A-L est mesurée dans le filtrat.

Solubilité des molécules de formule (L)-séryl-A-L

. Ester de l'hydroxyméthyl-3 benzoate d'hydroxyméthyl-4 biphényle de la L-sérine :
solubilité dans l'eau à 25 ° C : 0 g/l (insoluble)
. Ester de l'hydroxyméthyl-3 benzoate de benzyle de la L-sérine :
solubilité dans l'eau à 25 ° C : 0,2 g/l
. Ester de l'hydroxyméthyl-3 benzoate de phénoxy-3 benzyle de la L-sérine :
solubilité dans l'eau à 25 ° C : 0,75 g/l
A titre de comparaison :

. Ester méthylique de la L-sérine :
solubilité dans l'eau à 25 ° C : > 100 g/l
. Ester benzylique de la L-sérine :
solubilité dans l'eau à 25 ° C : > 100 g/l

V - EFFETS SOLUBILISANTS DANS LES SOLVANTS ORGANIQUES DU GROUPE LIPOPHILE PROTECTEUR DE LA FONCTION CARBOXYLIQUE DES PEPTIDES

L'effet solubilisant du groupe lipophile est mis en évidence par les mesures de solubilité décrites ci-après, où le peptide modèle est un peptide particulièrement hydrophile : la N-tertiobutyloxycarbonyl-pentaglycine

$(CH_3)_3-C-O-CO(NH-CH_2-CO)_5-OR$

| R | Solubilité en g/100 cm3 | | | Coefficient de partage entre CH2CL2 et H2O |
|---|---|---|---|---|
| | anisole | CH2Cl2 | H2O | |
| R1 | 5 | > 10 | 0,0016 | > 1.000 |
| R2 | 5 | > 10 | 0,0008 | > 2.000 |
| -CH3 * | insoluble | 0,33 | 0,50 | 0,66 |
| benzyle * | insoluble | 0,070 | 0,017 | 4,1 |

* à titre de comparaison

R1 =

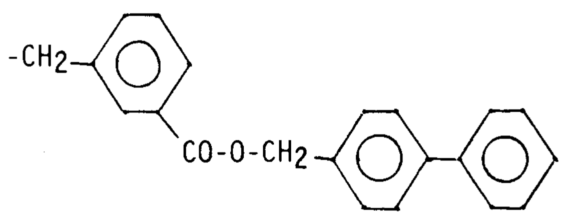

R2 =

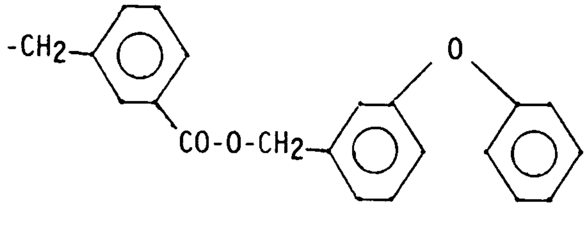

VI - SYNTHESE PEPTIDIQUE EN MILIEU ORGANIQUE CONCENTRE AU MOYEN DE GROUPES LIPOPHILES A-L

On synthétise le pentapeptide leucine encéphaline L-tyrosyl glycyl-glycyl-L-phénylalanyl-L-leucine :
- avec comme groupe lipophile A-L l'ester de méthyl-3 benzoate d'hydroxyméthyl-4 biphényle ;

- avec comme groupe protecteur de la fonction amine des acides aminés constitutifs le groupe tertiobutyloxycarbonyle.

Exemple 31

Exemple 31 a : Synthèse de l'ester de l'hydroxyméthyl-3 benzoate d'hydroxyméthyl-4 biphényle de la N-tertiobutyloxycarbonyl-L-phénylalanyl-L-leucine.

Dans un réacteur tricol de 500 $cm^3$, on introduit successivement :
- 10,33 g (0,039 mol) de N-tertiobutyloxycarbonyl-L-phénylalanine,
- 75 $cm^3$ de dichlorométhane.

Après dissolution, la température du milieu réactionnel est abaissée à -5°C et on ajoute successivement, sous agitation, 4,29 $cm^3$ de N-méthylmorpholine (0,039 mol), 4,46 $cm^3$ de chlorure de pivaloyle (0,036 mol).

Après 4 heures de réaction, on ajoute 14,018 g (0,030 mol) de chlorhydrate de l'ester de l'hydroxyméthyl-3 benzoate d'hydroxyméthyl-4 biphényle de la L-leucine, puis 3,3 $cm^3$ de M-méthylmorpholine (0,030 mol).

Après 2 heures de réaction, le mélange réactionnel est lavé successivement par :
2 x 25 $cm^3$ d'eau (pH 5,8 - 6,44),
3 x 30 $cm^3$ de $KHCO_3$ dilué (pH 8,2 - 8,3),
2 x 30 $cm^3$ d'eau (pH 7,3 - 7,2),
2 x 30 $cm^3$ de $H_2SO_4$ (pH 3,89 - 3,35),
2 x 30 $cm^3$ de NaCl dilué (pH 6,7 - 6,5).

Le rendement en produit dosé par rapport à un étalon est de 100 %.

L'analyse par chromatographie sur couche mince sur plaque de silice révèle une seule tache dans le système d'éluants suivant : acétate d'éthyle/hexane (2/5) Rf = 0,38.

La structure du produit a été vérifiée par spectrométrie de masse et par RMN du proton (360 MHz) et sa pureté a été confirmée par analyse par chromatographie liquide haute performance (CLHP).

Exemple 31 b : Synthèse du chlorhydrate de l'ester de l'hydroxyméthyl-3 benzoate d'hydroxyméthyl-4 biphényle de la L-phénylalanyl-L-leucine.

La solution obtenue après extraction dans l'exemple 31 a est séchée par distillation sous vide du dichlorométhane. Le volume final est ramené à 150 $cm^3$.

A température ambiante, on fait barboter dans la solution un courant d'acide chlorhydrique sec pendant 1 heure 45 minutes. Ensuite le mélange réactionnel est dégazé par bullage d'azote sec pendant 1 heure dans la solution.

Le mélange réactionnel est concentré sous vide jusqu'à un volume de 75 $cm^3$.

Une chromatographie en couche mince sur plaque de silice confirme la pureté du produit Rf = 0,8 dans le système d'éluants dichlorométhane/méthanol (90/10).

Une analyse par CLHP confirme que la coupure du groupe N-tertiobutyloxycarbonyle est quantitative (100 %).

Un échantillon de produit précipité dans le système acétate d'éthyle/éther éthylique présente un point de fusion de 167/169°C.

Exemple 31 c : Synthèse de l'ester de l'hydroxyméthyl-3 benzoate d'hydroxyméthyl-4 biphényle de la N-tertiobutyloxycarbonyl glycyl-glycyl -L-phénylalanyl-L-leucine.

Dans un réacteur tricol de 500 $cm^3$, on introduit successivement :
- 5,1 g (0,022 mol) de N-tertiobutyloxycarbonyl-glycyl-glycine,
- 75 $cm^3$ de dichlorométhane.

Après dissolution, la température du milieu réactionnel est abaissée à -5°C et on ajoute successivement, sous agitation, 2,4 $cm^3$ de M-méthylmorpholine (0,022 mol), 2,7 $cm^3$ de chlorure de pivaloyle (0,0176 mol).

Après 2 heures de réaction, sous agitation à-5°C, on ajoute la moitié de la solution obtenue dans l'exemple 31 b de chlorhydrate de l'ester de l'hydroxyméthyl-3 benzoate d'hydroxyméthyl-4 biphényle de la L-phénylalanyl-L-leucine (0,0147 mol dans 40 $cm^3$ de dichlorométhane), puis 1,62 $cm^3$ de N-méthylmorpholine (0,0147 mol).

Après 2 heures 30 minutes de réaction, sous agitation, le mélange réactionnel est lavé successivement par :

30 cm$^3$ de $H_2SO_4$ dilué (pH 3,5),
2 x 30 cm$^3$ d'eau (pH 4,5 -3,6),
2 x 30 cm$^3$ de NaOH diluée (pH 8,6 - 7,3),
2 x 30 cm$^3$ d'eau (pH 6,8 - 6,1),

L'analyse par chromatographie sur couche mince sur plaque de silice révèle un seul produit dans le système d'éluants suivant : dichlorométhane/méthanol (90/10) Rf 0,6.

La structure du produit a été vérifiée par spectrométrie de masse et par RMN du proton (360 MHz) et sa pureté a été confirmée par analyse par chromatographie liquide haute performance (CLHP).

Exemple 31 d : Synthèse du chlorhydrate de l'ester de méthyl-3 benzoate d'hydroxyméthyl-4 biphényle de la glycyl-glycyl-L-phénylalanyl-L-leucine.

La solution obtenue après extraction dans l'exemple 31 c est séchée par distillation sous vide du dichlorométhane. Le volume final est ramené à 75 cm$^3$.

A température ambiante, on fait barboter dans la solution un courant d'acide chlorhydrique sec pendant 1 heure 30 minutes. Ensuite le mélange réactionnel est dégazé par bullage d'azote sec pendant 1 heure dans la solution.

Le rendement en produit dosé de la solution ainsi obtenue est de 100 %.

La pureté du produit est vérifiée par chromatographie en couche mince sur plaque de silice et par CLHP.

Exemple 31 e : Synthèse de l'ester de (méthyl-3 benzoate d'hydroxyméthyl-4 biphényle) de la N-tertiobutyloxycarbonyl L-tyrosyl -glycyl-glycyl-L-phénylalanyl-L-leucine.

Dans un réacteur tricol de 250 cm$^3$, on introduit successivement :

- 5,26 g de N-tertiobutyloxycarbonyl-L-tyrosine,
- 40 cm$^3$ de dichlorométhane.

Après dissolution, la température du milieu réactionnel est abaissée à -15 °C et on ajoute successivement, sous agitation, 2,23 cm$^3$ de N-méthylmorpholine (0,02 mol), 2,5 cm$^3$ de chloroformiate d'isobutyle (0,019 mol).

Après 10 minutes de réaction, cette solution est ajoutée à la solution obtenue dans l'exemple 31 d de chlorhydrate de l'ester de méthyl-3 benzoate d'hydroxyméthyl-4 biphényle de la glycyl-glycyl-L-phénylalanyl-L-leucine (0,0135 moi dans 40 cm$^3$ de dichlorométhane), sous agitation à -15 °C puis on rajoute 1,5 cm$^3$ de N-méthylmorpholine (0,0135 mol).

Après 4 heures de réaction, sous agitation, le mélange réactionnel est lavé successivement par :

50 cm$^3$ de $H_2SO_4$ dilué (pH 6,6),
25 cm$^3$ de $H_2SO_4$ dilué (pH 2,2),
2 x 40 cm$^3$ d'eau (pH 4,3 -3,8),
40 cm$^3$ de $KHCO_3$ dilué (pH 8,1),
2 x 40 cm$^3$ d'eau (pH 7,9 - 6,9),

La phase organique est séchée par évaporation à sec pour donner 14,4 g d'un solide blanc d'aspect meringué.

L'analyse par chromatographie sur couche mince sur plaque de silice révèle un seul produit.

La structure du produit a été vérifiée par spectrométrie de masse et par RMN du proton (360 MHz) et sa pureté a été confirmée par analyse par chromatographie liquide haute performance (CLHP).

Exemple 31 f : Synthèse de la N-tertiobutyloxycarbonyl-L-tyrosyl-glycyl-glycyl-L-phénylalanyl-L-leucine.

Dans un ballon tricol de 100 cm$^3$, 5 g de l'ester de méthyl-3 benzoate d'hydroxyméthyl-4 biphényle de la N-tertiobutyloxycarbonyl-L-tyrosyl-glycyl-glycyl-L-phénylalanyl-L-leucine sont mis en suspension dans 15 cm$^3$ de méthanol.

On rajoute 15,7 cm$^3$ de soude N et 15 cm$^3$ d'acétonitrile.

Après 4 heures de réaction, on rajoute 20 cm$^3$ d'éther éthylique.

La phase aqueuse est décantée et lavée par 2 x 20 cm$^3$ d'éther éthylique, puis est acidifiée jusqu'à pH 3 par une solution de $KHSO_4$ à 5 %.

On extrait le N-tertiobutyloxycarbonylpentapeptide par le mélange acétate d'éthyle/éther éthylique 75/5.

La phase organique (75 $cm^3$) est lavée par 2 x 30 $cm^3$ d'eau (pH 4,0 - 5,0).

La phase organique finale est diluée à 100 $cm^3$ en fiole jaugée pour dosage par CLHP par rapport à un étalon de référence pur.

Le rendement dosé, après extraction et saponification, est de 94,5 % par rapport au chlorhydrate de l'ester de méthyl-3 benzoate d'hydroxyméthyl-4 biphényle de L-leucine, ce qui signifie que l'on a un rendement supérieur à 99 % pour chaque étape.

Après dosage la solution organique est concentrée et séchée sous très faible pression. Le résidu obtenu (3,5 g soit un rendement de 100 %) est dissous dans l'alcool isopropylique, puis est précipité par addition d'éther diisopropylique.

Cette opération est répétée une deuxième fois pour donner finalement 2,42 g d'une poudre, soit un rendement en produit isolé pur de 71 %.

La pureté du produit est vérifiée par analyse par CLHP, par spectrométrie RMN du proton (360 MHz) et par spectrométrie de masse.

Exemple 31 g : obtention du trifluoroacétate de la L-tyrosyl-glycyl-glycyl-L-phénylalanyl-L-leucine.

Dans un ballon tricol de 50 $cm^3$, on dissout à température ambiante 2,12 g (2,78 mmol) de N-tertiobutyloxycarbonyl-L-tyrosyl-glycyl-glycyl-L-phénylalanyl-L-leucine préparée dans l'exemple 31 f, dans 10 $cm^3$ d'un mélange 50/50 en volume de dichlorométhane/anisole et 5 $cm^3$ d'acide trifluoroacétique.

Après 1 heure de réaction sous agitation, la solution est concentrée sous pression réduite.

Le produit est dosé par rapport à un étalon de référence pur à 100 %.

Le rendement après coupure est de 100 % (2,78 mmol en produit dosé).

La solution dosée est ensuite lyophilisée pour donner 1,72 g d'une poudre blanche : rendement final en produit récupéré de 92,5 % par rapport à la N-tertiobutyloxycarbonyl-L-tyrosyl-glycyl-glycyl-L-phénylalanyl-L-leucine.

La pureté du produit est vérifiée par analyse par CLHP, par spectrométrie RMN du proton (360 MHz) et par spectrométrie de masse.

L'analyse élémentaire du produit final donne :

C = 53,1 %
H = 5,7 %
N = 9,9 %
F = 8,1 %

pour une formule brute :

$C_{28} H_{37} O_7 N_5, CF_3 COOH, 2H_2O$

**Revendications**

1. Procédé de solubilisation de peptides éventuellement protégés ou salifiés, dans un solvant organique non miscible à l'eau, caractérisé en ce que le groupe C-terminal dudit peptide est lié par l'intermédiaire d'une liaison amide ou ester à un groupe lipophile, ce dernier lorsqu'il est lié à la L-sérine donnant une molécule dont la solubilité dans l'eau à 25°C est inférieure à 30 g/litre, ce groupe lipophile étant chimiquement défini et non polymérique, le nombre de carbone dudit groupe lipophile étant supérieur à 7 et au plus égal à 50.

2. Procédé selon la revendication 1, caractérisé en ce que le groupe lipophile est composé de deux entités chimiques A et L liées entre elles par une liaison covalente dans lesquelles :
   - A représente un bras d'ancrage entre le peptide et le groupe L,
   - L représente un groupe hydrocarboné lipophile.

3. Procédé selon la revendication 2 caractérisé en ce que le groupe A est un bras d'ancrage bifonctionnel, comportant au moins une fonction (B) alcool ou amine et à l'autre extrémité une fonction carbonyle ou éther, et représenté par la formule générale :

   $B\text{-}R_1\text{-}Ar_{(k)}[R_2\text{-}(CO)_{(n)}\text{-}O_{(m)}]_p$

   dans laquelle :
   - B représente un groupe hydroxyle ou amino
   - Ar représente un radical aromatique mono ou polycyclique

- $R_1$ représente une liaison covalente, un radical alkylène contenant 1 à 4 atomes de carbone éventuellement substitué par un radical phényle, un groupe alkylènecarbonyle
- $R_2$ représente une liaison covalente, un radical alkylène contenant 1 à 4 atomes de carbone éventuellement substitué,
  un groupe oxyalkylène contenant 1 à 4 atomes de carbone dans la chaîne alkylène, un atome d'oxygène
- k, m et n sont des nombres entier égaux à 0 ou 1
- p est un nombre entier égal à 1 ou 2.

**4.** Procédé selon l'une des revendications 1 à 3 , caractérisé par le fait que ledit groupe lipophile est choisi de manière que la solubilité dans la phase organique soit au moins égal à 50 gramme par litre

**5.** Procédé selon la revendication 2, caractérisé en ce que les deux entités A et L peuvent former un seul groupe répondant à l'une des formules

$B - R_1 - Ar'$ ou $B - CH_{(3-q)} - Ar''_{(q)}$

dans lesquelles :
- B représente un groupe hydroxyle ou amino
- $R_1$ représente une liaison covalente, un radical alkylène contenant 1 à 4 atomes de carbone éventuellement substitué par un radical phényle, un groupe alkylènecarbonyle
- Ar' représente un radical aromatique polycyclique
- Ar'' représente un radical benzénique
- et q est un nombre entier égal à 2 ou 3.

**6.** Procédé de synthèse de peptides éventuellement protégés en milieu liquide, caractérisé en ce que l'on part d'un peptide ou d'un aminoacide initial solubilisé selon l'une quelconque des revendications 1 à 5 et en ce que l'on ajoute successivement un à un les aminoacides activés sur la fonction acide et protégés sur la fonction amine.

**7.** Procédé de synthèse de peptides éventuellement protégés en milieu liquide caractérisé, en ce que l'on part d'un aminoacide ou d'un peptide solubilisé selon l'une quelconque des revendications 1 à 5 et en ce que l'on condense un autre peptide ayant sa fonction acide C-terminale activée et sa fonction amine N-terminale protégée.

**8.** Procédé selon l'une quelconque des revendications 6 ou 7, caractérisé en ce que le milieu liquide est constitué d'un solvant choisi parmi les dérivés aliphatiques halogénés, les dérivés aromatiques et les esters.

**9.** Procédé selon l'une quelconque des revendications 6 à 8, caractérisé en ce que la fonction amine N-terminale est protégée par un groupe tertiobutyloxycarbonyle (Boc) ou par réaction avec un composé bétadicarbonylé.

**10.** Procédé selon l'une quelconque des revendications 6 à 8, caractérisé en ce que la fonction acide est activée par un chlorure d'acide, par un chloroformiate d'alkyle, par un carbodiimide ou par un ester activé.

**11.** Procédé selon l'une quelconque des revendications 6 à 10, caractérisé en ce que le peptide C-terminal initial est introduit dans le milieu réactionnel, puis l'acide aminé ou le peptide activé sur sa fonction acide et protégé sur sa fonction amine est ajouté, puis après réaction les co-produits et les excés de réactif sont éliminés de la phase organique par lavage avec des solutions aqueuses neutres, acides ou basiques, puis la fonction amine est libérée et enfin un nouvel acide aminé ou un nouveau peptide activé sur sa fonction acide et protégé sur sa fonction amine est introduit.

**12.** Réactif utile pour la synthèse peptidique caractérisé en ce qu'il consiste en une solution comportant un solvant non miscible à l'eau et au moins 50 gramme par litre d'un peptide éventuellement protégé et en ce que le groupe C-terminal dudit peptide est lié par l'intermédiaire d'une liaison amide ou ester à un groupe lipophile, ce dernier lorsqu'il est lié à la L-sérine donnant une molécule dont la solubilité

dans l'eau à 25°C est inférieure à 30 g/litre, ce groupe lipophile étant chimiquement défini et non polymérique, le nombre de carbone dudit groupe lipophile étant supérieur à 7 et au plus égal à 50.

## Claims

1. Process for the solubilization of peptides, optionally protected or salified, in a water-immiscible organic solvent, characterized in that the C-terminal group of the said peptide is linked via an amide or ester bond to a lipophilic group, the latter, when it is linked to L-serine, giving a molecule whose solubility in water at 25°C is less than 30 g/litre, this lipophilic group being chemically defined and non-polymeric, the carbon number of the said lipophilic group being greater than 7 and equal to not more than 50.

2. Process according to claim 1, characterized in that the lipophilic group is composed of two chemical entities A and L, linked to one another by a covalent bond, in which entities:
   - A represents an anchoring arm between the peptide and the group L,
   - L represents a lipophilic hydrocarbon group.

3. Process according to claim 2, characterized in that the group A is a bifunctional anchoring arm containing at least one alcohol or amine function (B) and at the other end a carbonyl or ether function, and represented by the general formula:

   $B\text{-}R_1\text{-}Ar_{(k)}[R_2\text{-}(CO)_{(n)}\text{-}O_{(m)}]_p$

   in which:
   - B represents a hydroxyl or amino group
   - Ar represents a mono- or polycyclic aromatic radical
   - $R_1$ represents a covalent bond, an alkylene radical containing 1 to 4 carbon atoms, optionally substituted with a phenyl radical, or an alkylenecarbonyl group
   - $R_2$ represents a covalent bond, an alkylene radical containing 1 to 4 carbon atoms, optionally substituted, an oxyalkylene group containing 1 to 4 carbon atoms in the alkylene chain or an oxygen atom
   - k, m and n are integers equal to 0 or 1
   - p is an integer equal to 1 or 2.

4. Process according to one of claims 1 to 3, characterized in that the said lipophilic group is chosen in such a way that the solubility in the organic phase is equal to at least 50 grams per litre.

5. Process according to claim 2, characterized in that the two entities A and L can form a single group corresponding to one of the formulae

   $B - R_1 - Ar'$ or $B - CH_{(3-q)} - Ar''_{(q)}$

   in which:
   - B represents a hydroxyl or amino group
   - $R_1$ represents a covalent bond, an alkylene radical containing 1 to 4 carbon atoms, optionally substituted with a phenyl radical, or an alkylene carbonyl group
   - Ar' represents a polycyclic aromatic radical
   - Ar'' represents a benzene radical
   - and q is an integer equal to 2 or 3.

6. Process for the synthesis of peptides, optionally protected, in a liquid medium, characterized in that the starting material is an initial peptide or amino acid solubilized according to any one of claims 1 to 5, and in that the amino acids, activated on the acid function and protected on the amine function, are added successively one by one.

7. Process for the synthesis of peptides, optionally protected, in a liquid medium, characterized in that the starting material is an amino acid or peptide solubilized according to any one of claims 1 to 5, and in that another peptide having its C-terminal acid function activated and its N-terminal amine function protected is condensed.

8. Process according to either of claims 6 and 7, characterized in that the liquid medium consists of a solvent selected from halogenated aliphatic derivatives, aromatic derivatives and esters.

9. Process according to any one of claims 6 to 8, characterized in that the N-terminal amine function is protected by a tert-butyloxycarbonyl (Boc) group or by reaction with a beta-dicarbonyl compound.

10. Process according to any one of claims 6 to 8, characterized in that the acid function is activated by an acid chloride, by an alkyl chloroformate, by a carbodiimide or by an activated ester.

11. Process according to any one of claims 6 to 10, characterized in that the initial C-terminal peptide is introduced into the reaction medium, the amino acid or peptide activated on its acid function and protected on its amine function is then added, the by-products and the excess reactant are then removed from the organic phase after reaction by washing with neutral, acidic or basic solutions, the amine function is then liberated and finally a further amino acid or a further peptide activated on its acid function and protected on its amine function is introduced.

12. Reactant which is useful for peptide synthesis, characterized in that it consists of a solution containing a water-immiscible solvent and at least 50 grams per litre of an optionally protected peptide, and in that the C-terminal group of the said peptide is linked via an amide or ester bond to a lipophilic group, the latter, when it is linked to L-serine, giving a molecule whose solubility in water at 25°C is less than 30 g/litre, this lipophilic group being chemically defined and non-polymeric, the carbon number of the said lipophilic group being greater than 7 and equal to not more than 50.

**Patentansprüche**

1. Verfahren zur Solubilisierung von gegebenenfalls geschützten oder als Salze vorliegenden Peptiden in einem organischen,
mit Wasser nicht mischbaren Lösemittel,
dadurch gekennzeichnet,
daß die C-terminale Gruppe des Peptids durch das Bindeglied einer Amid- oder Esterbindung an eine lipophile Gruppe gebunden ist, wobei diese letztere, wenn sie an L-Serin gebunden ist, ein Molekül ergibt, dessen Löslichkeit in Wasser bei 25°C weniger als 30 g/l beträgt, diese lipophile Gruppe chemisch definiert und nicht polymer ist und die Anzahl der Kohlenstoffatome dieser lipophilen Gruppe mehr als 7 und höchstens 50 beträgt.

2. Verfahren nach Anspruch 1,
dadurch gekennzeichnet,
daß die lipophile Gruppe aus zwei chemischen Einheiten A und L zusammengesetzt ist, welche miteinander durch eine kovalente Bindung verbunden sind, in welchen:
   - A eine verankernde Gruppierung zwischen dem Peptid und der Gruppe L darstellt, und
   - L eine lipophile Kohlenwasserstoffgruppe darstellt.

3. Verfahren nach Anspruch 2,
dadurch gekennzeichnet,
daß die Gruppe A eine bifunktionale verankernde Gruppe darstellt, welche mindestens eine Alkohol- oder Aminfunktion (B) und an dem anderen Ende eine Carbonyl- oder Etherfunktion trägt, und durch die allgemeine Formel:

$$B\text{-}R_1\text{-}Ar_{(k)}[R_2\text{-}(CO)_{(n)}\text{-}O_{(m)}]_p$$

dargestellt wird,
in welcher:
   - B eine Hydroxyl- oder Aminogruppe darstellt,
   - Ar einen aromatischen, mono- oder polycyclischen Rest darstellt,
   - $R_1$ eine kovalente Bindung, einen gegebenenfalls durch einen Phenylrest substituierten Alkylenrest mit 1 bis 4 Kohlenstoffatomen oder eine Alkylencarbonylgruppe darstellt,
   - $R_2$ eine kovalente Bindung, einen gegebenenfalls substituierten Alkylenrest mit 1 bis 4 Kohlenstoffatomen, eine Oxyalkylengruppe mit 1 bis 4 Kohlenstoffatomen in der Alkylenkette oder ein

Sauerstoffatom darstellt,
- k, m und n ganze Zahlen gleich 0 oder 1 sind, und
- p eine ganze Zahl gleich 1 oder 2 ist.

**4.** Verfahren nach einem der Ansprüche 1 bis 3,
dadurch gekennzeichnet,
daß die lipophile Gruppe so ausgewählt wird, daß die Löslichkeit in der organischen Phase mindestens 50 g/l beträgt.

**5.** Verfahren nach Anspruch 2,
dadurch gekennzeichnet,
daß die zwei Einheiten A und L eine einzelne Gruppe bilden können, welche einer der Formeln

$B - R_1 - Ar'$ oder $B - CH_{(3-q)} - Ar''_{(q)}$

entspricht,
in welchen:
- B eine Hydroxyl- oder Aminogruppe darstellt,
- $R_1$ eine kovalente Bindung, einen gegebenenfalls durch einen Phenylrest substituierten Alkylenrest mit 1 bis 4 Kohlenstoffatomen oder eine Alkylencarbonylgruppe darstellt,
- Ar' einen aromatischen polycyclischen Rest darstellt,
- Ar'' einen Benzolrest darstellt,
- und q eine ganze Zahl gleich 2 oder 3 darstellt.

**6.** Verfahren zum Herstellen von gegebenenfalls geschützten Peptiden in flüssiger Phase,
dadurch gekennzeichnet,
daß man von einem Starterpeptid oder einer Starteraminosäure ausgeht, welches bzw. welche nach einem der Ansprüche 1 bis 5 solubilisiert worden ist, und nacheinander hinsichtlich deren Säuregruppe aktivierte und hinsichtlich deren Aminogruppe geschützte Aminosäuren einzeln zugibt.

**7.** Verfahren zum Herstellen von gegebenenfalls geschützten Peptiden in flüssiger Phase,
dadurch gekennzeichnet,
daß man von einer Aminosäure oder einem Peptid ausgeht, welche bzw. welches nach einem der Ansprüche 1 bis 5 solubilisiert worden ist, und diese(s) mit einem anderen Peptid, dessen C-terminale Säuregruppe aktiviert und dessen N-terminale Aminogruppe geschützt ist, kondensiert.

**8.** Verfahren nach einem der Ansprüche 6 oder 7,
dadurch gekennzeichnet,
daß die flüssige Phase aus einem Lösemittel besteht, welches ausgewählt ist aus halogenierten aliphatischen Verbindungen, aromatischen Verbindungen und Estern.

**9.** Verfahren nach einem der Ansprüche 6 bis 8,
dadurch gekennzeichnet,
daß die N-terminale Aminogruppe durch eine tert. -Butyloxycarbonylgruppe (BOG-Gruppe) oder durch eine Reaktion mit einer $\beta$-Dicarbonylverbindung geschützt ist.

**10.** Verfahren nach einem der Ansprüche 6 bis 8,
dadurch gekennzeichnet,
daß die Säuregruppe durch ein Säurechlorid, ein Alkylchlorformiat, ein Carbodiimid oder einen aktivierten Ester aktiviert wird.

**11.** Verfahren nach einem der Ansprüche 6 bis 10,
dadurch gekennzeichnet,
daß das C-terminale Starterpeptid in das Reaktionsmedium eingeführt wird, dann die oder das hinsichtlich seiner Säuregruppe aktivierte und hinsichtlich seiner Aminogruppe geschützte Aminosäure oder Peptid zugegeben wird, nach der Reaktion dann die Nebenprodukte und die überschüssigen Reagenzien aus der organischen Phase durch Waschen mit wäßrigen, neutralen, sauren oder basischen Lösungen entfernt werden, dann die Aminofunktion freigesetzt wird und zuletzt eine neue oder

ein neues, hinsichtlich seiner Säuregruppe aktivierte(s) und hinsichtlich seiner Aminogruppe geschützte(s) Aminosäure oder Peptid eingeführt wird.

**12.** Reagens, welches für eine Peptidsynthese verwendet werden kann, dadurch gekennzeichnet, daß es aus einer Lösung umfassend ein mit Wasser nicht mischbares Lösemittel und mindestens 50 g/l eines gegebenenfalls geschützten Peptids besteht und daß die C-terminale Gruppe des Peptids durch das Bindeglied einer Amid- oder Esterbindung an eine lipophile Gruppe gebunden ist, wobei diese letztere, wenn sie an L-Serin gebunden ist, ein Molekül ergibt, dessen Löslichkeit in Wasser bei 25 °C weniger als 30 g/l beträgt, diese lipophile Gruppe chemisch definiert und nicht polymer ist und die Anzahl der Kohlenstoffatome dieser lipophilen Gruppe mehr als 7 und höchstens 50 beträgt.